# EUROPEAN PATENT APPLICATION

(11) **EP 1 396 255 A1**
(43) Date of publication of application: **10.03.2004**
(21) Application number: 02700812.7
(22) Date of filing: 26.02.2002
(51) Int. Cl.: A61K 7/00, A61K 7/48, A61K 7/40, A61K 35/78, A61K 45/00, A61P 5/30, A61P 17/00

(54) **METHOD FOR SUPPRESSING REDUCTION OF ELASTICITY OF SKIN**

(30) Priority: 26.02.2001 JP 2001050839
(71) Applicant: SHISEIDO COMPANY, LTD., Chuo-ku, Tokyo 104-8010 (JP)
(72) Inventor: OCHIAI, N. SHISEIDO RESEARCH CNTR(SHIN-YOKOHAMA), Yokohama-shi, kanagawa 224-8558 (JP); INOMATA, S. SHISEIDO RESEARCH CNTR(SHIN-YOKOHAMA), Yokohama-shi, Kanagawa 224-8558 (JP); TAKADA, K. SHISEIDO RESEARCH CNTR(SHIN-YOKOHAMA), Yokohama-shi, Kanagawa 224-8558 (JP)
(74) Representative: Santarelli
(86) International application number: PCT/JP2002/001757
(87) International publication number: WO 2002/067873

(57) **Abstract**

The present invention provides a preparation, for preventing a decrease in skin elasticity, comprising a matrix metalloproteinase inhibitor as an active ingredient and also a method of preventing a decrease in skin elasticity using the same.

## Description

### FIELD OF THE INVENTION

The present invention relates to a preparation for preventing skin aging, and particularly skin sagging, due to reduced female hormones associated with ovarian insufficiency, particularly reduced female hormones during menopause. More specifically, the present invention relates to a preparation for preventing skin aging due to reduced female hormones associated with ovarian insufficiency, said preparation comprising, as an active ingredient, a substance having a potent inhibitory action on the activity of matrix metalloproteinases.

### BACKGROUND ART

Conventionally, it has been reported that female hormones drastically decrease in the post-menopausal period, which is accompanied by aggravated aging of the skin resulting in increased "sagging" (Journal of the Japan Menopause Society, Vol. 8, pp. 33-40, 2000). As one of the causes for this, a reduction in collagen of the skin in the post-menopausal period has been reported (Maturitus 15: 113-119 (1992); Br. J. Obstet. Gynecol. 100: 154-156 (1993)). However, as the mechanism of collagen reduction during menopause has not been elucidated, no effective means has been found to prevent it other than the administration of female hormones. The latter method is not practical, however, because the administration of female hormones has a risk of causing side effects, and thereby formulation comprising the same is greatly limited. Thus, there has been a great need for substances, that effectively suppress aging phenomena such as "sagging" which becomes specifically pronounced during menopause, as anti-aging agents during menopause.

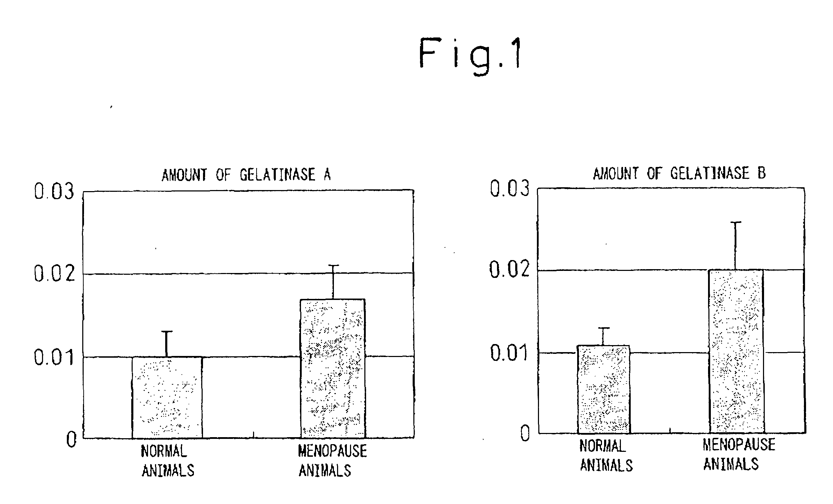

In efforts to elucidate the mechanism involved in aging changes in the skin in the post-menopausal period, specifically "sagging", the present inventors have used academically recognized ovarectomized mice as a menopause model, and made intensive study on the mechanism of onset of skin aging accompanied by "sagging" etc. As a result, the cause of "sagging" that develops in the post-menopausal period was narrowed down to the enhanced expression of matrix metalloproteinases, in particular, gelatinase belonging to the matrix metalloproteinase, in the skin due to decreased female hormones in the skin in the post-menopausal period (see Fig. 1).

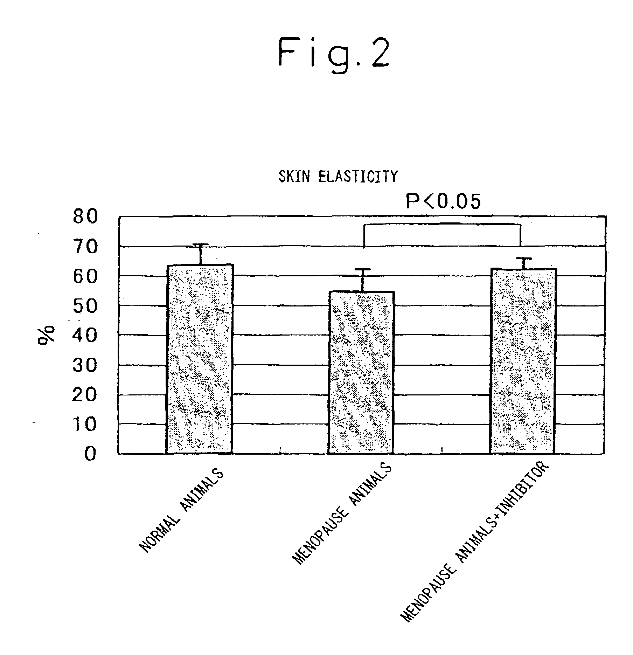

Matrix metalloproteinases are well known to be zinc-containing proteolytic enzymes that hydrolyze extracellular matrix-forming proteins including collagen (Seikagaku 71(12): 1387-1401 (1999)), and to cause changes due to the aging of the skin, i.e. wrinkles, darkeness, loss of surface texture, and the like (see Japanese Unexamined Patent Publication (Kokai) No. 2000-256122). However, the enhanced expression was mainly attributed to UV irradiation (Gary J. Fischer et al., Nature 379(25), 335(1996); Gary J. Fischer et al., The New England Journal of Medicine, 337(20), 1419(1997)), and the association of reduction in female hormones and the enhanced expression of matrix metalloproteinases was not known. The present inventors have found that reduction in female hormones in the post-menopausal period is accompanied by the increase in this enzyme, which causes enhanced decomposition of extracellular matrix-forming proteins of the skin, resulting in increased "sagging." And when a substance that inhibits matrix metalloproteinase was externally applied to the mouse menopause model, "sagging" that increases by reduction in female hormones is markedly suppressed (see Fig. 2). Thus, the present inventors have found that matrix metalloproteinase inhibitors are effective for the prevention and improvement of skin aging processes such as "sagging" that increase due to reduced female hormones in the post-menopausal period, and thereby have accomplished the present invention.

### DISCLOSURE OF THE INVENTION

Thus, the present invention relates to a preparation for preventing skin aging due to reduced female hormones associated with ovarian insufficiency, said preparation comprising, as an active ingredient, a matrix metalloproteinase inhibitor.

In one aspect, the above reduction in female hormones associated with ovarian insufficiency is caused by menopause.

In a further aspect, the above aging of the skin comprises the sagging of the skin.

In a further aspect, the above matrix metalloproteinase is a protease belonging to the gelatinase group.

The present invention further relates to a method of suppressing skin aging, said method comprising applying the above preparation for preventing skin aging to the skin.

In accordance with the present invention, the active ingredient of the preparation for preventing skin aging is not specifically limited as long as it inhibits matrix metalloproteinase, and, for example, N-hydroxy-2(R)[(4-methoxyphenyl)sulfonyl](3-picolyl)-methyl butanamide (referred to as active ingredient A), or a salt thereof, for example a hydrochloride thereof, which belongs to hydroxamic derivatives, is preferred. As used herein, the structure of the hydrochloride of active ingredient A is shown by the following formula:

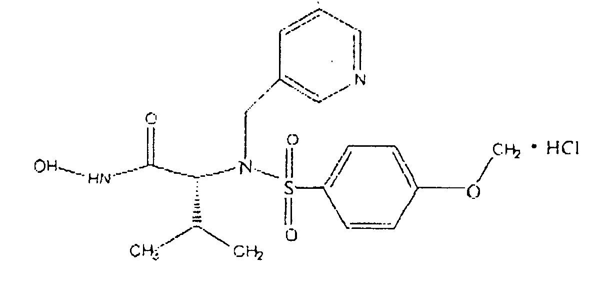

Substances that inhibit matrix metalloproteinase other than hydroxamic acid derivatives may include doxycycline having a tetracycline backbone, as natural compounds, curcumine for which an excellent gelatinase inhibiting effect was found and plant extracts (turmeric extract) containing it, as well as a plant extract mangosteen (Garcinia mangostana L.), DaunDuduk, for which the matrix metalloproteinase inhibiting effect has been confirmed.

Mangosteen (Garcinia mangostana L.) mentioned above is a plant belonging to the family Guttiferae (Hypericaceae)) Genus Garcinia. It is an evergreen fruiter, and the peel portion contains tannin and a yellow'dye (mangosteen). A dry bark provides a black pigment.

For mangosteen (G. mangostana), preferably the peel or the bark is used, but other parts can also be used.

Extracts of the above plants can be obtained by standard methods, and for example, they can be obtained by immersing in an extraction solvent or heating to reflux each of the above plants, after which they can be filtered and concentrated to obtain the extracts. As the extraction solvent, any commonly used ones may be used, and for example water, alcohols such as methanol, ethanol, propylene glycol, 1,3-butylene glycol and glycerin, organic solvents such as water-containing alcohols, chloroform, dichloroethane, carbon tetrachloride, acetone, ethyl acetate and hexane may be used alone or in combination. The extracts as they are obtained by extracting with the above solvents, or those obtained by removing impurities from the concentrated extract with an adsorption method or an ion exchange resin, those obtained by allowing them adsorbed onto a column of porous polymer (for example Amberlite XAD-2) and then eluting with methanol or ethanol followed by concentration can also be used. Extracts etc. obtained by a partition method, for example extraction with water/ethyl acetate can also be used.

As used herein, there are known many types of matrix metalloproteinases (Kaoru Miyazaki, Seikagaku 6(12): 1791-1807 (1996)). Matrix metalloproteinases (MMP) include, for example, MMP1 that degrades type I and III collagen which are major skin matrix-building components ; MMP2 (gelatinase A), MMP9 (gelatinase B) belonging to the gelatinase group that degrades type IV collagen and laminin which are components of basal membrane and elastin which is a dermis matrix-component; MMP3 and MMP10 belonging to the stromelysin group that degrades proteoglycans, type IV collagen, laminin etc., and the like.

The preparation for preventing skin aging according to the present invention is particularly effective for the prevention and improvement of skin aging, particularly the sagging of the skin, due to reduced female hormones associated with ovarian insufficiency. Ovarian insufficiency associated with reduced female hormones include various ovarian insufficiencies accompanied by the depression of the female hormone-producing function of the ovary, such as climacteric disturbance and ovarian deficiency symptoms. Also, female hormones include estrogenic hormones (estrogen), for example estradiol, estrin, estriol, corpus luteum hormone (progesterone) and the like.

"Skin aging due to reduced female hormones associated with ovarian insufficiency" as used herein means changes in the skin resulting from increased matrix metalloproteinases in the skin, for example reduced elasticity of the skin, in particular wrinkles, loss of texture, sagging of the skin, and particularly sagging.

The degree of "sagging" as used herein can be judged by determining mainly the elasticity of the skin, and can be determined by a method known to a person skilled in the art. For example, elasticity can be determined by measuring the ratio (%) of the skin recovering after being extended at a given pressure. In this case, the higher the degree of recovering the higher elasticity the skin has, and thus judged to have smaller "sagging." The measurement of such a ratio of recovering can be measured using, for example, the skin elasticoviscosity measuring instrument Cutometer (manufactured by COURAGE KHAZAKSA).

### BRIEF EXPLANATION OF THE DRAWINGS

Fig. 1 is a graph showing a relationship between ovarian insufficiency and the amount of a matrix metalloproteinase.
Fig. 2 is a graph showing the effect of matrix metalloproteinase inhibitor of the present invention on the sagging of the skin.

### BEST MODE FOR CARRYING OUT THE INVENTION

The construction of the present invention will now be explained in more detail.

That the preparation for preventing skin aging for use in the present invention containing a matrix metalloproteinase inhibitor exhibits an effect of preventing skin aging due to reduced female hormones associated with ovarian insufficiency, for example the effect of preventing skin aging during menopause, was found for the first time by the present inventors. Accordingly, it was found that the preparation for preventing skin aging for use in the present invention containing a matrix metalloproteinase inhibitor is particularly effective for preventing and improving the sagging of the skin during menopause.

The amount blended of the matrix metalloproteinase inhibitor in the preparation for preventing skin aging of the present invention is 0.0001-20.0% by mass, preferably 0.0001-10.0% by mass in terms of dry weight in the total amount of the preparation for preventing skin aging. when the amount is less than 0.0001% by mass, the effect mentioned in the present invention cannot be fully exhibited, and when it exceeds 20.0% by mass, it is difficult to formulate into drugs or the like and thus is undesirable. Furthermore, the blending of the amount over 10.0% by mass does not result in significant increases in efficacy.

In addition to the above-mentioned essential ingredients, the preparation for preventing skin aging containing matrix metalloproteinase inhibitor of the present invention may be blended, as appropriate, with ingredients used in external preparations such as conventional cosmetics and pharmaceutical preparations, including for example whitening agents, moisturizers, antioxidants, oily components, UV absorbers, surfactants, thickners, alcohols, powder components, coloring agents, aqueous components, water, various skin nutrients, and the like, as needed.

In addition, there may be blended, as appropriate, metal blocking agents such as edetate disodium, edetate trisodium, sodium citrate, sodium polyphosphate, sodium metaphosphate, and gluconic acid, caffeine, tannin, verapamil, tranexamic acid and derivatives thereof, licorice extracts, glabridin, hot water extracts of padauk fruit, various crude drugs, tocopherol acetate, drugs such as glycyrrhetic acid and derivatives thereof or salts thereof, vitamin C, ascorbic acid magnesium phosphate, ascorbic acid glucoside, arbutin, other whitening agents such as kojic acid, sugars such as glucose, fructose, mannose, sucrose and trehalose, vitamin A such as retinoic acid, retinol, retinol acetate, and retinol palmitate, and the like.

The preparation for preventing skin aging containing matrix metalloproteinase inhibitor of the present invention may be formulated to any form that are conventionally used in drugs for external application to the skin such as ointments, creams, emulsions, lotions, packs, and baths, and the dosage forms are not specifically limited.

### EXAMPLES

The present invention will now be explained in more detail with reference to examples. It should be noted that the present invention is not limited by these examples. The amount blended is expressed in % by weight.

### Experiment 1. Relationship between ovarian insufficiency and the amount of matrix metalloproteinase

Female hairless mice were subjected to bilateral ovarectomy, and used as an animal model of menopause (ovarian insufficiency). Mice that were not subjected to such ovarectomy were used as the normal animals. Two months after ovary removal, the skin was extracted from the back of the female hairless mice, subcutaneous tissue thereof was removed, and divided into the epidermis and the dermis after heated in hot water at 60°C. The dermis was extracted with 0.05M Tris-HCl buffer, centrifuged (8000×g, Hitachi), and the amount of gelatinase in the centrifugation supernatant was analyzed by a gelatin zymography. Numerical expression of the amount of gelatinase was carried out using the Flour-S Multi Imager (BIORAD) by analyzing the intensity of bands obtained by the gelatin zymography. The amount of gelatinase in the skin of the normal animals was also measured according to a similar protocol.

As can be seen from the result of Fig. 1, the amount of gelatinase A and B in the skin increased in the animal model of menopause compared to the normal animals. Thus, this demonstrates that the extraction of the female hormone-producing ovary results in an increase in the amount of gelatinase belonging to the matrix metalloproteinase in the skin.

### Experiment 2. Experiment on the effect of suppressing reduction in skin elasticity due to reduced female hormones

### (1) Skin elasticity experiment

Five-week old female hairless mice were subjected to bilateral ovarectomy, and used as an animal model of menopause. For two months from immediately after ovary extraction, 100 µl of 1% inhibitor (hydrochloride of active ingredient A) in 80% ethanol, 15% of 1,3-butylene glycol and 5% dynamite glycerin was applied onto the back five times per week. As a control, a group (menopause animals + inhibitor) in which the solvent of the inhibitor was applied in a similar protocol and the normal group (normal animals) that did not receive ovarectomy were set up.

Two months after ovary removal, skin elasticity of the back was measured. A skin section was pulled by a given pressure (200 mPa) for five seconds using Cutometer (COURAGE KHAZAKSA), and then the ratio of the skin section returning was measured as recovery to determine the mean and the standard deviation for each group. It was judged that the greater the value is, the more elasticity, and hence less "sagging", there is.

### (2) Result

As can be seen from Fig. 2, the matrix metalloproteinase inhibitor suppresses a decrease in skin elasticity due to reduced female hormones and suppressed "sagging" in the post-menopausal period, and thus an effect of preventing skin aging was noted, which indicates that the blending of a matrix metalloproteinase-suppressing agent in drugs for external use as an active ingredient of a preparation for preventing skin aging during menopause is a very useful regimen.

### Experiment 3. Preparation of plant extracts and test on inhibitory effect on the activity of MMPs

### [Testing method and evaluation method)

### 1. Preparation of samples

### (1) Plant extracts

As shown in Table 1, each plant was immersed in methanol at room temperature for 1 week to obtain an extract. This extract was concentrated to yield each plant extract (methanol extract).

**Table 1**

| Plant name | Part | Amount of plant used (g) | Amount of solvent (methanol) used (ml) | Yield of plant extract (g) |
|---|---|---|---|---|
| Mangosteen (Garcinia mangostana) | Peel | 200 | 450 | 1.52 |
| Mangosteen (Garcinia mangostana) | Bark | 200 | 500 | 3.01 |

### (2) Sample solution

Plant extract was dissolved at a concentration of 2% by weight in dimethyl sulfoxide (DMSO) to prepare a plant extract-containing solution.

The plant extract-containing solution was diluted in an assay buffer (0.1 M Tris, pH 7.4, containing 0.4 M NaCl, 10 mM CaCl₂), and the concentration was adjusted as shown in Table 2, which was used as a sample solution and was subjected to the following experiment.

### 2. Test on inhibitory effect of MMPs activity (in vitro) inhibition ratio of MMP-9 activity

As an enzyme belonging to the gelatinase group, MMP-9 was used, and the inhibition ratio of its activity was determined. The measurement was carried out as follows.

SDS-polyacrylamide gel (10% T) containing 0.2% by weight gelatin was prepared, and a given amount of human cell-derived MMP-9 solution was applied in all lanes and then was subjected to electrophoresis. The gel after electrophoresis was washed in 2.5% by mass solution of Triton X-100, and then in an incubation buffer (0.50 M Tris, pH 8.0, containing 0.01 mM ZnSO₄, 5 mM CaCl₂) to sufficiently remove SDS. Gel was cut into strips, and each cut gel was immersed in the incubation buffer to which a given amount of MMP-9 was added, and then incubated overnight at 37°C.

After incubation, the gel was stained with Coomassie Brilliant Blue, and the widths of the bands appearing after destaining were quantitated using an image analyzer (manufactured by BIORAD [Fluor-SMulti Imager]).

A reduction of the band width in a system containing the plant extract (the above sample solution) relative to the band width in a system containing no plant extract (control, DMSO) was determined, and MMP-9 activity inhibition ratio (%) was calculated. The result is shown in Table 2.

**Table 2**

| Sample | Concentration of sample solution (% by weight) | Enzyme | Inhibition of MMPs activity(%) |
|---|---|---|---|
| Mangosteen (Garcinia mangostana) (peel) | 0.0005 | MMP-9 | 68 |
| Mangosteen (Garcinia mangostana) (peel) | 0.005 | MMP-9 | 98 |
| Mangosteen (Garcinia mangostana) (bark) | 0.0005 | MMP-9 | 71 |
| Mangosteen (Garcinia mangostana) (bark) | 0.005 | MMP-9 | 100 |

### 3. Study on in vivo effect

The plant extract-containing sample solution was investigated concerning the inhibitory effect on decrease in skin elasticity of the menopause model (ovarectomized mice, non-UV irradiated).

After removing the ovaries from 6-week old female mice, 100 µl of the plant extract-containing sample solution was applied five times per week for 12 weeks. The result demonstrated that the plant extract-containing sample solution improves the skin elasticity of the menopause model.

Formulation examples of active ingredient A in various dosage forms according to the present invention are explained as working Examples below.

### Example 1. Cream

| (Formulation) | |
|---|---|
| Stearic acid | 5.0 % by mass |
| Stearyl alcohol | 4.0 |
| Isopropyl myristate | 18.0 |
| Glycerin monostearate | 3.0 |
| Propylene glycol | 10.0 |
| Hydrochloride of active ingredient A | 1.0 |
| Caustic potash | 0.2 |
| Sodium bisulfite | 0.01 |
| Preservative | q.s. |
| Flavor | q.s. |
| Ion exchanged water | Balance |

### (Method of preparation)

To ion exchanged water, propylene glycol, active ingredient A and caustic potash are added and dissolved, and then heated and maintained at 70°C (aqueous phase). The other ingredients are mixed, molten under heating and maintained at 70°C (oily phase). The oily phase is gradually added to the aqueous phase, and after the total amount has been added, the temperature is maintained for a while in order to allow the reaction to occur. It is then emulsified to homogeneity by a homomixer, and cooled to 30°C with sufficient stirring.

### Example 2. Cream

| (Formulation) | |
|---|---|
| Stearic acid | 2.0 % by mass |
| Stearyl alcohol | 7.0 |
| Hydrogenated lanolin | 2.0 |
| Squalane | 5.0 |
| 2-octyldodecyl alcohol | 6.0 |
| Polyoxyethylene (25 mole) cetylalcohol ether | 3.0 |
| Glycerin monostearate | 2.0 |
| Propylene glycol | 5.0 |
| Hydrochloride of active ingredient A | 0.05 |
| Sodium bisulfite | 0.03 |
| Ethylparaben | 0.3 |
| Flavor | q.s. |
| Ion exchanged water | Balance |

### (Method of preparation)

To ion exchanged water, propylene glycol is added, and heated and maintained at 70°C (aqueous phase). The other ingredients are mixed, melted by heating and maintained at 70°C (oily phase). The oily phase is added to the aqueous phase to pre-emulsify, and after emulsified to homogeneity by a homomixer, it is cooled to 30°C with sufficient stirring.

### Example 3. Cream

| (Formulation) | |
|---|---|
| Solid paraffin | 5.0 % by mass |
| Beeswax | 10.0 |
| Vaseline | 15.0 |
| Liquid paraffin | 41.0 |
| Glycerin monostearate | 2.0 |
| Polyoxyethylene (20 mole) sorbitan monolaurate | 2.0 |
| Soap powder | 0.1 |
| Borax | 0.2 |
| Hydrochloride of active ingredient A | 0.01 |
| Sodium bisulfite | 0.03 |
| Ethylparaben | 0.3 |
| Flavor | q.s. |
| Ion exchanged water | Balance |

### (Method of preparation)

To ion exchanged water, soap powder and borax are added, dissolved under heating, and maintained at 70°C (aqueous phase). The other ingredients are mixed, melted by heating, and maintained at 70°C (oily phase). The oily phase is gradually added to the aqueous phase to allow the reaction to occur. After the reaction is complete, it is emulsified to homogeneity by a homomixer, and after emulsification it is cooled to 30°C with sufficient stirring.

### Example 4. Lotion

| (Formulation) | |
|---|---|
| Stearic acid | 2.5 % by mass |
| Cetyl alcohol | 1.5 |
| Vaseline | 5.0 |
| Liquid paraffin | 10.0 |
| Polyoxyethylene (10 mole) | |
| monooleate | 2.0 |
| Polyethylene glycol 1500 | 3.0 |
| Triethanolamine | 1.0 |
| Carboxyvinyl polymer | 0.05 |
| (Trade name: Carbopol 941, B.F. Goodrich Chemical Company) | |
| Hydrochloride of active ingredient A | 0.01 |
| Sodium bisulfite | 0.01 |
| Ethylparaben | 0.3 |
| Flavor | q.s. |
| Ion exchanged water | Balance |

### (Method of preparation)

In a small amount of ion exchanged water, carboxyvinyl polymer is dissolved (phase A). To the rest of ion exchanged water, polyethylene glycol 1500 and triethanolamine are added, dissolved by heating, and maintained at 70°C (aqueous phase). The other ingredients are mixed, molten under heating, and maintained at 70°C (oily phase). The oily phase is added to the aqueous phase to pre-emulsify, to which phase A is added and emulsified to homogeneity by a homomixer. After emulsification, it is cooled to 30°C with sufficient stirring.

### Example 5. Lotion

| (Formulation) | |
|---|---|
| Macrocrystalline wax | 1.0 % by mass |
| Beexwax | 2.0 |
| Lanoline | 20.0 |
| Liquid paraffin | 10.0 |
| Squalane | 5.0 |
| Sorbitan sesquioleate | 4.0 |
| Polyoxyethylene (20 mole) sorbitan monooleate | 1.0 |
| Propylene glycol | 7.0 |
| Hydrochloride of active ingredient A | 10.0 |
| Sodium bisulfite | 0.01 |
| Ethylparaben | 0.3 |
| Flavor | q.s. |
| Ion exchanged water | Balance |

### (Method of preparation)

To ion exchanged water, propylene glycol is added, and heated and maintained at 70°C (aqueous phase). The other ingredients are mixed, molten under heating, and maintained at 70°C (oily phase). While the oily phase is stirred, the aqueous phase is gradually added thereto, and emulsified to homogeneity by a homomixer. After emulsification, it is cooled to 30°C with sufficient stirring.

### Example 6. Jelly

| (Formulation) | |
|---|---|
| 95% ethyl alcohol | 10.0 % by mass |
| Dipropylene glycol | 15.0 |
| Polyoxyethylene (50 mole) oleylalcahol ether | 2.0 |
| Carboxyvinyl polymer | 1.0 |
| (Trade name: Carbopol 940, B.F. Goodrich Chemical Company) | |
| Caustic soda | 0.15 |
| L-arginine | 0.1 |
| Hydrochloride of active ingredient A | 7.0 |
| Sodium 2-hydroxy-4-methoxybenzophenone sulfonate | 0.05 |
| Ethylenediaminetetraacetic acid trisodium dihydrate | 0.05 |
| Methylparaben | 0.2 |
| Flavor | q.s. |
| Ion exchanged water | Balance |

### (Method of preparation)

In ion exchanged water, Carbopol 940 is homogeneously dissolved, while the hydrochloride of active ingredient A and polyoxyethylene (50 mole) oleylalcohol ether are dissolved and added to the aqueous phase. Then, the other ingredients are added, and caustic soda and L-arginine are added to neutralize it and to increase viscosity.

### Example 7. Cosmetic liquid

| (Formulation) | |
|---|---|
| (Phase A) | |
| Ethyl alcohol (95%) | 10.0 % by mass |
| Polyoxyethylene (20 mole) octyldodecanol | 1.0 |
| Panthothenilethyl ether | 0.1 |
| Hydrochloride of active ingredient A | 1.5 |
| Methylparaben | 0.15 |

| (Phase B) | |
|---|---|
| Potassium hydroxide | 0.1 |

| (Phase C) | |
|---|---|
| Glycerin | 5.0 |
| Dipropylene glycol | 10.0 |
| Sodium bisulfite | 0.03 |
| Carboxyvinyl polymer | 0.2 |
| (Trade name: Carbopol 940, B.F. Goodrich Chemical Company) | |
| Purified water | Balance |

### (Method of preparation)

Phase A and phase B are separately dissolved, and phase A is added to phase C. Then phase B is added thereto and filling is performed.

### Example 8. Pack

| (Formulation) | |
|---|---|
| (Phase A) | |
| Dipropylene glycol | 5.0 % by mass |
| Polyoxyethylene (60 mole) hydrogenated castor oil | 5.0 |

| (Phase B) | |
|---|---|
| Hydrochloride of active ingredient A | 0.01 |
| Olive oil | 5.0 |
| Tocopherol acetate | 0.2 |
| Ethyl parabene | 0.2 |
| Flavor | 0.2 |

| (Phase C) | |
|---|---|
| Sodium bisulfite | 0.03 |
| Polyvinyl alcohol | 13.0 |
| (degree of saponification 90, degree of polymerization 2,000) | |
| Ethanol | 7.0 |
| Purified water | Balance |

### (Method of preparation)

Phase A, phase B and phase C are separately dissolved to homogeneity, and phase A is added to phase B to solubilize. Then this is added to phase C and filling is performed.

### Example 9. Solid Foundation

| (Formulation) | |
|---|---|
| Talc | 43.1 % by mass |
| Kaolin | 15.0 |
| Cericite | 10.0 |
| zinc flower | 7.0 |
| Titanium dioxide | 3.8 |
| Yellow iron oxide | 2.9 |
| Black iron oxide | 0.2 |
| Squalane | 8.0 |
| Isostearic acid | 4.0 |
| Monooleic acid POE sorbitan | 3.0 |
| Isocetyl Octoate | 2.0 |
| Hydrochloride of active ingredient A | 1.0 |
| Preservative | q.s. |
| Flavor | q.s. |

### (Method of preparation)

Powder components of talc to black iron oxide are mixed well in the blender, and then oily components of squalane to isocetyl octaoate, the hydrochloride of active ingredient A, a preservative and a flavor are added thereto, and after blending well, it is filled into a container and molded.

### Example 10. Emulsified Foundation (cream type)

| (Formulation) | |
|---|---|
| (Powder portion) | |
| Titanium dioxide | 10.3 % by mass |
| Cericite | 5.4 |
| Kaolin | 3.0 |
| Yellow iron oxide | 0.8 |
| Red oxide | 0.3 |
| Black iron oxide | 0.2 |

| (oily phase) | |
|---|---|
| Decamethylcyclopentasiloxane | 11.5 |
| Liquid paraffin | 4.5 |
| Polyoxyethylene denatured Dimethylpolysiloxane | 4.0 |

| (aqueous phase) | |
|---|---|
| Purified water | 50.0 |
| 1,3-butyleneglycol | 4.5 |
| Hydrochloride of active ingredient A | 1.5 |
| Sorbitan sesquioleate | 3.0 |
| Preservative | q.s. |
| Flavor | q.s. |

### (Method of preparation)

After the aqueous phase is stirred under heating, a fully mixed and ground powder portion is added thereto and treated by a homomixer. After the heated and mixed oily phase is further added and treated by a homomixer, a flavor is added with stirring, and then cooled to room temperature.

### Example 11. Cream

| (Formulation) | |
|---|---|
| Stearic acid | 5.0 % by mass |
| Stearyl alcohol | 4.0 |
| Isopropyl myristate | 18.0 |
| Glycerin monostearate | 3.0 |
| Propylene glycol | 10.0 |
| Mangosteen (bark) | 1.0 |
| Caustic potash | 0.2 |
| Sodium bisulfite | 0.01 |
| Preservative | q.s. |
| Flavor | q.s. |
| Ion exchanged water | Balance |

### (Method of preparation)

To ion exchanged water, propylene glycol, active ingredient A and caustic potash are added and dissolved, and then heated and maintained at 70°C (aqueous phase). The other ingredients are mixed, molten under heating and maintained at 70°C (oily phase). The oily phase is gradually added to the aqueous phase, and after the total amount has been added, the temperature is maintained for a while in order to allow the reaction to occur. It is then emulsified to homogeneity by a homomixer, and cooled to 30°C with sufficient stirring.

### Example 12. Lotion

| (Formulation) | |
|---|---|
| Stearic acid | 2.5 % by mass |
| Cetyl alcohol | 1.5 |
| Vaseline | 5.0 |
| Liquid paraffin | 10.0 |
| Polyoxyethylene (10 mole) monooleate | 2.0 |
| Polyethylene glycol 1500 | 3.0 |
| Triethanolamine | 1.0 |
| Carboxyvinyl polymer | 0.05 |
| (Trade name: Carbopol 941, B.F. Goodrich Chemical Company) | |
| Mangosteen (bark) | 0.01 |
| Sodium bisulfite | 0.01 |
| Ethylparaben | 0.3 |
| Flavor | q.s. |
| Ion exchanged water | Balance |

### (Method of preparation)

To a small amount of ion exchanged water, carboxyvinyl polymer is dissolved (phase A). To the rest of ion exchanged water, polyethylene glycol 1500 and triethanolamine are added, dissolved under heating, and maintained at 70°C (aqueous phase). The other ingredients are mixed, molten under heating, and maintained at 70°C (oily phase). The oily phase is added to the aqueous phase to pre-emulsify, to which phase A is added and emulsified to homogeneity by a homomixer. After emulsification, it is cooled to 30°C with thorough stirring.

### Example 13. Pack

| (Formulation) | |
|---|---|
| (Phase A) | |
| Dipropylene glycol | 5.0 % by mass |
| polyoxyethylene (60 mole) hydrogenated castor oil | 5.0 |

| (Phase B) | |
|---|---|
| Daun Duduk | 0.01 |
| Olive oil | 5.0 |
| Tocopherol acetate | 0.2 |
| Ethyl parabene | 0.2 |
| Flavor | 0.2 |

| (Phase C) | |
|---|---|
| Sodium bisulfite | 0.03 |
| Polyvinyl alcohol | 13.0 |
| (degree of saponification 90, degree of polymerization 2,000) | |
| Ethanol | 7.0 |
| Purified water | Balance |

### (Method of preparation)

Phase A, phase B and phase C are separately dissolved to homogeneity, and phase A is added to phase B to solubilize. Then this is added to phase C and filling is performed.

### Industrial Applicability

The preparation for preventing skin aging containing matrix metalloproteinase inhibitor of the present invention exhibits the effect of inhibiting skin aging due to reduced female hormones associated with ovarian insufficiency such as menopause and, in particular, acts favorably in preventing and/or improving "sagging" etc. in the post-menopausal period.

## Claims

1. A method of preventing a decrease in skin elasticity due to reduced female hormones associated with ovarian insufficiency, comprising using a matrix metalloproteinase inhibitor as an active ingredient.

2. The method of preventing a decrease in skin elasticity according to claim 1 wherein a condition in which female hormones are decreased associated with ovarian insufficiency is menopause.

3. The method of preventing a decrease in skin elasticity according to claim 1 or 2 wherein said decrease in skin elasticity is the sagging of the skin.

4. The method of preventing a decrease in skin elasticity according to any one of claims I to 3 wherein said matrix metalloproteinase is a protease belonging to the gelatinase group.

5. The method of preventing a decrease in skin elasticity according to any one of claims 1 to 4, comprising applying said matrix metalloproteinase inhibitor as an active ingredient to the skin.

6. A method of preventing a decrease in skin elasticity due to reduced female hormones associated with ovarian insufficiency, comprising using a mangosteen extract as a matrix metalloproteinase inhibitor.
